(19) Europäisches Patentamt / European Patent Office / Office européen des brevets

(11) **EP 4 039 314 A1**

(12) **EUROPEAN PATENT APPLICATION**
published in accordance with Art. 153(4) EPC

(43) Date of publication:
**10.08.2022 Bulletin 2022/32**

(21) Application number: **19948046.8**

(22) Date of filing: **01.10.2019**

(51) International Patent Classification (IPC):
***A61M 25/01*** *(2006.01)*

(52) Cooperative Patent Classification (CPC):
**A61M 25/01**

(86) International application number:
**PCT/JP2019/038703**

(87) International publication number:
**WO 2021/064853 (08.04.2021 Gazette 2021/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Japan Lifeline Co., Ltd.
Tokyo 140-0002 (JP)**

(72) Inventor: **NIWA Yuki
Tokyo 140-0002 (JP)**

(74) Representative: **Stöckeler, Ferdinand et al
Schoppe, Zimmermann, Stöckeler
Zinkler, Schenk & Partner mbB
Patentanwälte
Radlkoferstrasse 2
81373 München (DE)**

(54) **CATHETER**

(57) The present invention provides a catheter including a novel strain relief that prevents or suppresses kinks of a catheter body. An electrode catheter 100 including a hollow tube-shaped strain relief 200 that includes a hollow part 210 penetrating through in an axial direction, and a catheter body 10 having a base end part 10a inserted into the hollow part is provided, in which the strain relief has an outer-diameter gradually-decreasing part 230 toward a tip end, and the gradual decrease rate of the outer diameter in the outer-diameter gradually-decreasing part increases toward the tip end. The strain relief 200 includes at least two gradient change parts 241 and 242 where the gradual decrease rate of the outer diameter changes. In the strain relief 200, the inner diameter of the hollow part 210 gradually decreases from the base end to the tip end. The strain relief 200 is particularly suitable for a catheter body 10 having an outer diameter equal to or less than 1.0 millimeter.

FIG.3

EP 4 039 314 A1

**Description**

Field

The present invention relates to various catheters such as an electrode catheter and a balloon catheter.

Background

A catheter such as an electrode catheter or a balloon catheter includes a catheter body to be inserted into a blood vessel of a human body, and a handle for holding the catheter body.

The catheter body is formed of a flexible material that can be curved or deformed according to the shape of a blood vessel of a human body. Meanwhile, the handle for operating the catheter body is formed of a material having a higher hardness than that of the catheter body. Stress concentration is likely to occur on a coupling portion of the catheter body to the handle and portions of the catheter body located near the coupling portion due to the difference in the hardness between the catheter body and the handle, and kinks (bends) are prone to occur. If the catheter body kinks, the operation force does not appropriately transmit to the catheter body. As a result, a problem occurs in which an insertion operation and a removal operation of the catheter body become difficult.

The catheter generally includes a strain relief at the coupling portion between the handle and the catheter body for adjustment to reduce the stress concentration occurring between the handle and the catheter body, thereby preventing or suppressing kinks of the catheter body. Patent Literature 1 describes an electrode catheter including a strain relief.

Citation List

Patent Literature

Patent Literature 1: Japanese Patent Application Laid-open No. 2018-143603

Summary

Technical Problem

The strain relief disclosed in Patent Literature 1 has a relatively general shape including an outer diameter that gradually decreases toward a tip end at a constant gradual decrease rate. Accordingly, Patent Literature 1 does not disclose any specific configuration for effectively preventing or suppressing kinks of the catheter body.

The present invention has been achieved in view of the circumstances described above and has an object to provide a catheter including a novel strain relief that prevents or suppresses kinks of a catheter body.

Solution to Problem

In order to solve the above problems, a catheter according to the present invention comprises a hollow tube-shaped strain relief including a hollow part penetrating through in an axial direction, and a catheter body having a base end part inserted into the hollow part, wherein the strain relief includes an outer-diameter gradually-decreasing part in which an outer diameter thereof gradually decreases toward a tip end, and a gradual decrease rate of the outer diameter in the outer-diameter gradually-decreasing part increases toward the tip end.

Advantageous Effects of Invention

According to the present invention, it is possible to provide a catheter including a novel strain relief that prevents or suppresses kinks of a catheter body.

Brief Description of Drawings

[FIGS. 1] FIGS. 1 are diagrams illustrating an electrode catheter to which a strain relief according to one embodiment of the present invention is applied, where (a) is a side view and (b) is a partially enlarged sectional view.
[FIG. 2] FIG. 2 is a perspective view illustrating one of divided pieces of a handle.
[FIG. 3] FIG. 3 is a vertical sectional view illustrating a strain relief according to a first embodiment of the present invention.

[FIG. 4] FIG. 4 is a vertical sectional view illustrating a strain relief according to a second embodiment of the present invention.

Description of Embodiments

The present invention will be described below in detail with embodiments illustrated in the drawings. Constituent elements, types, combinations, shapes, and relative arrangements described in the embodiments are merely explanatory examples, and are not intended to limit the scope of the present invention solely thereto unless otherwise specified.

[Electrode catheter]

Strain reliefs according to embodiments of the present invention are used in various catheters such as an electrode catheter and a balloon catheter. A schematic configuration of an electrode catheter as an example of catheters to which the strain reliefs according to the embodiments of the present invention are applied is explained below.

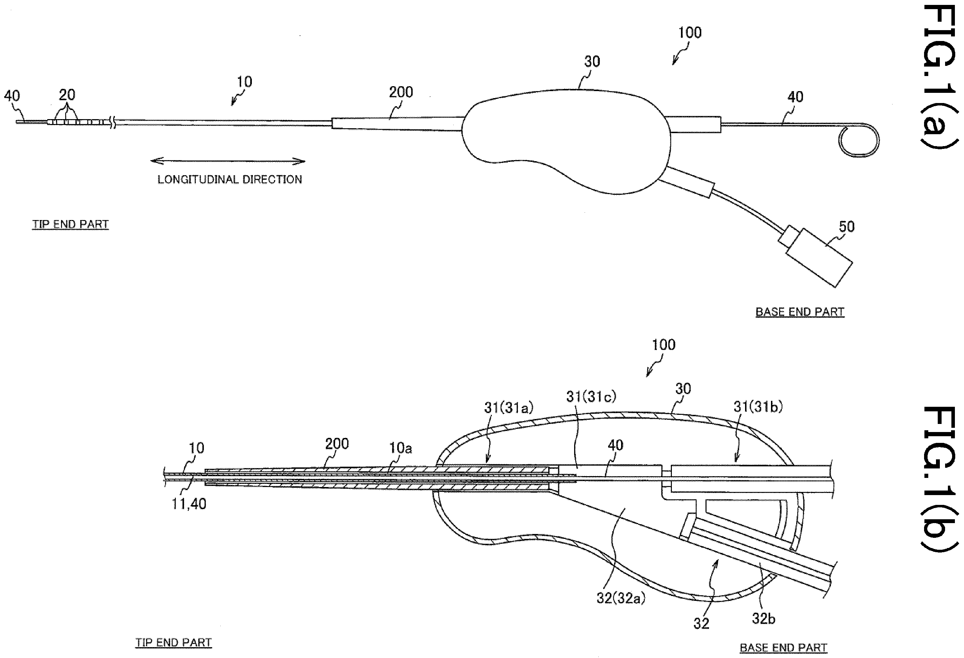

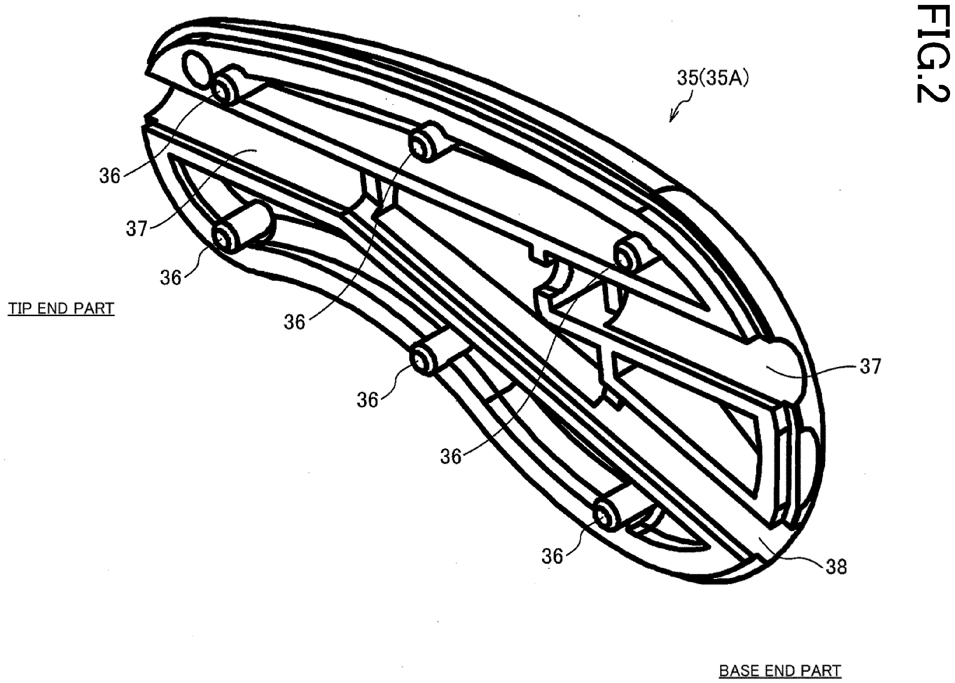

FIGS. 1 are diagrams illustrating an electrode catheter to which a strain relief according to one embodiment of the present invention is applied, where (a) is a side view and (b) is a partially enlarged sectional view. FIG. 2 is a perspective view illustrating one of divided pieces of a handle.

An electrode catheter (catheter) 100 is an instrument to be inserted into a heart through a blood vessel and used in mapping of electrical activity states in the heart or cardiac potential measurement after ablation (cauterization) of an inner wall of the heart. The electrode catheter described in the present example is an over-the-wire (OTW) electrode catheter.

The electrode catheter 100 includes a catheter body 10 extending in a longitudinal direction, a plurality of ring-shaped electrodes 20, 20, ⋯ attached on a tip end part (a distal end side) of the catheter body 10, and a handle 30 attached to a base end part 10a (a proximal end side) of the catheter body 10. The handle 30 supports the base end part 10a of the catheter body 10 inserted into a strain relief 200.

<Catheter body>

The catheter body 10 is constituted of a tubular member including a central lumen 11 extending in the longitudinal direction and a plurality of sub lumens (not illustrated) arranged circumferentially around the central lumen 11. A guide wire 40 guiding the catheter body 10 to a required position in a human body is inserted into the central lumen 11. The guide wire 40 can move back and forth in the central lumen 11 along the longitudinal direction. Lead wires (not illustrated) being respectively conductive with the ring-shaped electrodes 20, 20, ⋯ are inserted in the sub lumens.

The catheter body 10 is formed of a flexible material. Specifically, synthetic resins such as a polyolefin, a polyamide, a polyetherpolyamide, a polyurethane, a nylon, and PEBAX (registered trademark, material name: polyether block amide) can be used for the catheter body 10.

<Handle>

The handle 30 described in the present example is used in a catheter (an electrode catheter or a balloon catheter). The handle 30 is formed of a non-flexible material having a higher hardness than that of the catheter body 10. Examples of the non-flexible material are synthetic resins such as an ABS resin, a polycarbonate, and a polyethylene.

The handle 30 is constituted of a pair of divided pieces 35 that are obtained by division into two along a virtual division plane (a plane parallel to the plane of paper of FIG. 1(a)) extending along the longitudinal direction of the catheter body 10. FIG. 2 illustrates only one divided piece 35A. The divided pieces have substantially symmetric shapes.

The divided piece 35A includes a plurality of coupling protrusions 36 protruded in a direction orthogonal to the division plane, and the other divided piece includes coupling recesses that respectively receive the coupling protrusions 36. By fitting the coupling protrusions 36 of the divided piece 35A in the coupling recesses of the other divided piece, the both divided pieces are unitized to form the handle 30.

As illustrated in FIG. 1(b), the handle 30 includes a first through hole 31 penetrating through from the tip end to the base end. The first through hole 31 is formed linearly in the handle 30. The handle 30 includes a second through hole 32 branching off from an intermediate portion in the longitudinal direction of the first through hole 31. One tip end 32a in the longitudinal direction of the second through hole 32 is communicated with the first through hole 31 at an intermediate portion 31c (the branch portion) in the longitudinal direction of the first through hole 31 and the other end 32b in the longitudinal direction of the second through hole 32 is open at an appropriate position on the base end side of the handle 30.

As illustrated in FIG. 2, a first recess 37 and a second recess 38 both having a half-column shape are formed on each of the divided pieces 35. The first recess 37 and the second recess 38 respectively form the first through hole 31 and the second through hole 32 with the other divided piece.

The first through hole 31 includes a support part 31a at a tip end part, which supports the base end part 10a of the catheter body 10. The first through hole 31 includes a guide wire path 31b at a base end part, which guides the guide wire 40 into the central lumen 11 of the catheter body 10. In a case in which the handle 30 is used for the electrode catheter 100, the second through hole 32 functions as a lead wire path for drawing out the lead wires in the catheter 10 to outside. The lead wires drawn outside from the catheter body 10 are conductively connected to an electrode connector 50.

In a case in which the handle 30 is used for a balloon catheter, the second through hole 32 functions as a path that introduces a fluid for expanding a balloon arranged at the tip end part of the catheter body.

A portion of the catheter body 10 covered by the strain relief 200 is housed in the support part 31a of the handle 30.

The handle 30 is used as a common member to catheters that use the catheter bodies 10 having different outer diameter sizes. That is, the handle 30 is enabled to be used as a common member to the catheter bodies 10 having different outer diameter sizes by adapting the outer diameter of the strain relief 200 to a size according to the shape of the support part 31a and the inner diameter of the strain relief 200 to a size according to the outer diameter of the catheter body 10.

[Strain relief]

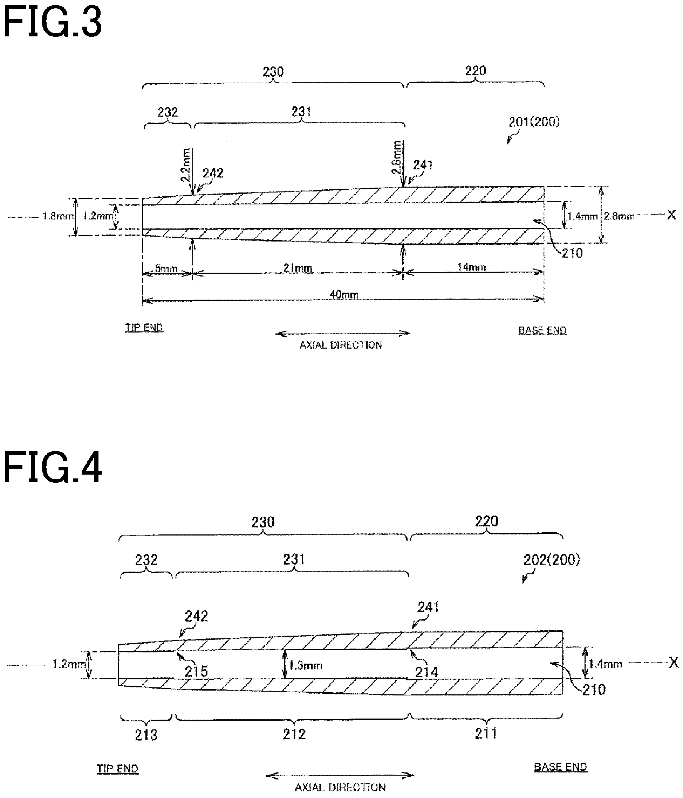

FIG. 3 is a vertical sectional view illustrating a strain relief according to a first embodiment of the present invention. The strain relief according to the present embodiment is characterized in including a region where the outer diameter gradually decreases from the base end to the tip end and in that the inner diameter gradually decreases from the base end to the tip end.

A strain relief 201 (200) has a hollow tube shape, more specifically, a hollow cylindrical shape having a hollow part 210 penetrating through in an axial direction. The base end part 10a of the catheter body 10 (see FIG. 1(b)) is inserted into the hollow part 210. The strain relief 201 reduces stress concentration occurring between the handle 30 and the catheter body 10. The strain relief 201 has a function to prevent or suppress kinks at the base end part of the catheter body 10 inserted into the hollow part 210.

<Outer shape>

The strain relief 201 has an outer diameter unchanged part 220 where the outer diameter is uniform (the gradual decrease rate of the outer diameter is zero) on a base end side, and an outer-diameter gradually-decreasing part 230 of a tapered shape where the outer diameter gradually decreases toward the tip end on a tip end side.

The base end side of the strain relief 201 is housed in the handle 30 and the remaining part (the tip end side) is exposed from the handle 30. At least a portion of the outer diameter unchanged part 220 is a housed part that is housed in the support part 31a of the handle 30. The whole of the outer-diameter gradually-decreasing part 230 of the strain relief 201 is an exposed part that is exposed from the handle 30. The gradual decrease rate (the gradient or the inclination rate with respect to an axis line X) of the outer diameter of the outer-diameter gradually-decreasing part 230 is configured to increase from the base end to the tip end.

The gradual decrease rate of the outer diameter is a value indicating the degree of decrease of the outer diameter per unit length along the axial direction. When the outer diameters at certain two points are D1 and D2, and the axial distance between these two points is LD, the gradual decrease rate DR (%) of the outer diameter between the two points is represented by

$$DR = \{(D1 - D2)/LD\} \times 100 \qquad \text{Expression (1)}$$

(where it is assumed that the point of the outer diameter D1 is positioned nearer the base end than the point of the outer diameter D2).

Furthermore, a state in which the gradual decrease rate of the outer diameter in the outer-diameter gradually-decreasing part 230 increases from the base end to the tip end indicates a state in which a relation

$$DRb \geq DRa \qquad \text{Expression (2)}$$

is met between a gradual decrease rate DRa of the outer diameter between certain two points set on a side near the base end and a gradual decrease rate DRb of the outer diameter between different certain two points set on a side nearer the tip end than the aforementioned two points. While there may be cases in which "DRb=DRa" holds depending on setting of certain two points, "DRb<DRa" never holds. A position where "DRb>DRa" holds needs to be always included in the outer-diameter gradually-decreasing part 230.

The outer-diameter gradually-decreasing part 230 includes a first outer-diameter gradually-decreasing part 231 that is located on the base end side and that has the outer diameter changing at a first gradual decrease rate, and a second outer-diameter gradually-decreasing part 232 that is located on the tip end side and that has the outer diameter changing at a second gradual decrease rate larger than the first gradual decrease rate. In the present example, the first gradual decrease rate and the second gradual decrease rate have fixed values, respectively.

The first gradual decrease rate is desirably set to be not less than 20% and not more than 40%. The second gradual decrease rate is desirably set to be not less than 70% and not more than 90%. The difference between the second gradual decrease rate and the first gradual decrease rate is desirably set to be equal to or more than 30%.

The strain relief 201 has two gradient change parts 241 and 242 where the gradual decrease rate of the outer diameter changes. That is, the strain relief 201 has a first gradient change part 241 where the gradual decrease rate of the outer diameter changes between (a connection point) the outer diameter unchanged part 220 and the first outer-diameter gradually-decreasing part 231, and has a second gradient change part 242 where the gradual decrease rate of the outer diameter changes between (a connection point) the first outer-diameter gradually-decreasing part 231 and the second outer-diameter gradually-decreasing part 232. Any step (portion where the outer shape changes in a stepwise manner) is not formed on the outer circumferential surface of the strain relief 201.

The outer-diameter gradually-decreasing part 230 may have three or more regions where the gradual decrease rates of the outer diameter are different. The strain relief 201 may include three or more gradient change parts in the axial direction.

<Inner shape>

The hollow part 210 of the strain relief 201 has a tapered shape with the inner diameter gradually decreasing from the base end to the tip end. The strain relief 201 does not have steps in the hollow part 210. The inner diameter of the strain relief 201 decreases at a fixed gradual decrease rate from the base end to the tip end. The gradual decrease rate of the inner diameter is a value indicating the degree of decrease of the inner diameter per unit length along the axial direction. When the inner diameters at certain two points are d1 and d2, and the axial distance between these two points is Ld, the gradual decrease rate dR (%) of the inner diameter between the two points is represented by

$$dR = \{(d1-d2)/Ld\} \times 100 \qquad \text{Expression (3)}$$

(where it is assumed that the point of the inner diameter d1 is positioned nearer the base end than the point of the inner diameter d2).

The gradual decrease rate of the inner diameter is desirably set to be not less than 1% and not more than 30%.

The catheter body 10 having the outer diameter that is uniform in the axial direction is inserted into the hollow part 210 having the tapered shape.

The inner shape of the strain relief 201 is a simplified shape where the gradual decrease rate of the inner diameter is uniform entirely in the axial direction. Meanwhile, since the outer diameter of the outer diameter unchanged part 220 is uniform, the wall thickness of the outer diameter unchanged part 220 increases toward the tip end. The maximum wall thickness required for the outer-diameter gradually-decreasing part 230 is ensured at the first gradient change part 241, which is the connection point between the outer-diameter gradually-decreasing part 230 and the outer diameter unchanged part 220.

The gradual decrease rate of the outer diameter in the outer-diameter gradually-decreasing part 230 (the first outer-diameter gradually-decreasing part 231 and the second outer-diameter gradually-decreasing part 232) is larger than the gradual decrease rate of the inner diameter in the outer-diameter gradually-decreasing part 230. Therefore, the wall thickness of the outer-diameter gradually-decreasing part 230 reduces toward the tip end.

<Dimension example>

A dimension example of the catheter body 10 and the strain relief 201 is as follows.

The outer diameter (diameter) of the catheter body 10 to be inserted into the hollow part 210 of the strain relief 201 is 2.7 Fr (=0.9 millimeter (mm), where 1 Fr=0.33 mm).

The entire length (axial length) of the strain relief 201 is not less than 30 mm and not more than 100 mm.

FIG. 3 illustrates an example of the strain relief 201 having the entire length of 40 mm. In the example of FIG. 3, the axial length of the outer diameter unchanged part 220 is 14 mm, the axial length of the first outer-diameter gradually-decreasing part 231 is 21 mm, and the axial length of the second outer-diameter gradually-decreasing part 232 is 5 mm.

The outer diameter of the outer diameter unchanged part 220 (from the base end to the first gradient change part 241) is 2.8 mm, the outer diameter of the second gradient change part 242 is 2.2 mm, and the outer diameter of the tip end is 1.8 mm. The inner diameter of the base end is 1.4 mm and the inner diameter of the tip end is 1.2 mm.

The dimension example of the strain relief 201 described above provides values of the strain relief 201 as a unit component. When the strain relief 201 is assembled to the catheter body 10, the parts of the strain relief 201 may have different values from those described above.

The axial length and the outer shape of the outer diameter unchanged part 220 are set according to the axial length and the inner shape of the support part 31a of the handle 30.

The wall thickness of the outer-diameter gradually-decreasing part 230 is preferably set between 0.2 and 3.0 mm.

The axial length of the second outer-diameter gradually-decreasing part 232 is preferably set between 10% and 40%, more specifically between 20% and 30% with respect to the axial length of the outer-diameter gradually-decreasing part 230.

<Manufacturing method and material>

The strain relief 201 is formed, for example, using a styrene elastomer as its material. The strain relief 201 is molded, for example, by injection molding. Since the strain relief 201 according to the present embodiment has a fixed gradual decrease rate of the inner diameter, a mold for injection molding can be easily formed. Since the inner diameter of the strain relief 201 gradually decreases toward the tip end, the components of the strain relief 201 can be easily removed from the mold.

The constituent material and the manufacturing method of the strain relief 201 described above are merely an example and the strain relief 201 may be formed of other materials and by other manufacturing methods.

The handle 30 is constituted, for example, using an ABS resin as its material.

The catheter body 10 is fixed to the strain relief 201 with an adhesive. The strain relief 201 is fixed to the handle 30 with an adhesive. While a two-component curable epoxy resin adhesive can be used as the both adhesives, other types of adhesives may be used.

<Effect>

The strain relief 201 according to the present embodiment is effective as means for preventing or suppressing kinks of an ultrafine catheter body 10 with the diameter being particularly about equal to or less than 3 Fr (=1.0 mm). However, the strain relief 201 described in the present embodiment also effectively functions as means for preventing or suppressing kinks of a thicker catheter body (for example, about 6 Fr: 2.0 mm or more).

Among catheter bodies, particularly an ultrafine catheter body 10 of about equal to or less than 3 Fr is likely to have a large hardness difference from the handle and how to prevent or suppress kinks becomes an issue. If the wall thickness of the strain relief is reduced to be adapted to the hardness of the ultrafine catheter body 10, occurrence of break, split, or the like of the strain relief caused by the wall thickness reduction presents a problem. If the wall thickness of the strain relief is made too thin, molding becomes difficult and this leads to deterioration in the fabrication yield.

Since the gradual decrease rate of the outer diameter of the second outer-diameter gradually-decreasing part 232 is set to be higher than that of the first outer-diameter gradually-decreasing part 231 in the strain relief 201 according to the present embodiment, the axial length of the second outer-diameter gradually-decreasing part 232 having a relatively small wall thickness can be shortened. Therefore, damages such as break or split of the strain relief 201 caused by the wall thickness reduction can be effectively prevented and the molding is facilitated.

With use of the strain relief 201 according to the present embodiment, the catheter body 10 having a relatively small outer diameter (for example, about 3 Fr) can be attached to the handle 30 adapted to a catheter body having a relatively large outer diameter (for example, about 6 Fr). That is, in the strain relief 201 according to the present embodiment, not only the outer shape of the outer diameter unchanged part 220 is adapted to the inner shape of the support part 31a of the handle 30 but also at least two gradient changing parts where the gradual decrease rate of the outer diameter changes are formed to change the gradual decrease rate of the outer diameter in a stepwise manner. Furthermore, the inner diameter of the strain relief 201 is configured to gradually decrease in the axial direction, so that the handle 30 can be used as a common component to the electrode catheters 100 using the catheter bodies 10 having different outer diameter sizes.

In the present embodiment, since the inner diameter of the hollow part 210 is gradually decreased from the base end to the tip end, the decrease of the wall thickness in the outer-diameter gradually-decreasing part 230 is enabled to be gentle. Therefore, damages such as break or split of the strain relief caused by the wall thickness reduction can be effectively prevented while the wall thickness required for the outer-diameter gradually-decreasing part 230 of the strain relief 201 is ensured.

In the outer-diameter gradually-decreasing part 230, the gradual decrease rate of the outer diameter is larger than that of the inner diameter and therefore the wall thickness of the outer-diameter gradually-decreasing part 230 is gradually thinned toward the tip end. As compared to a strain relief in which the gradual decrease rate of the outer diameter is same as that of the strain relief according to the present embodiment and the inner diameter is uniform in the axial direction, the decrease of the wall thickness in the axial direction is gentler in the present embodiment in which the inner diameter gradually decreases in the axial direction. Since the hardness changes are also gentle according to the degree of decrease of the wall thickness of the outer-diameter gradually-decreasing part 230 in the present embodiment, kinks of the catheter body 10 can be prevented or suppressed more effectively.

[Second embodiment]

FIG. 4 is a vertical sectional view illustrating a strain relief according to a second embodiment of the present invention. The strain relief according to the present embodiment is characterized in that the inner diameter of the hollow part gradually decreases in a stepwise manner from the base end to the tip end. Differences between the second embodiment and the first embodiment are mainly explained below. Parts identical to those of the first embodiment are denoted by like reference signs and explanations thereof are appropriately omitted.

The hollow part 210 of a strain relief 202 (200) includes a base-end-side hollow part 211, an intermediate hollow part 212, and a tip-end-side hollow part 213 in this order from the base end side. The inner diameter of the intermediate hollow part 212 is smaller than that of the base-end-side hollow part 211, and the inner diameter of the tip-end-side hollow part 213 is smaller than that of the intermediate hollow part 212.

A first stepped part 214 where the inner diameter discontinuously changes is formed between (a connection point) the base-end-side hollow part 211 and the intermediate hollow part 212. A second stepped part 215 where the inner diameter discontinuously changes is formed between (a connection point) the intermediate hollow part 212 and the tip-end-side hollow part 213.

Each of the inner diameters of the base-end-side hollow part 211, the intermediate hollow part 212, and the tip-end-side hollow part 213 is uniform in the axial direction. For example, the inner diameter of the base-end-side hollow part 211 can be set to 1.4 mm, the inner diameter of the intermediate hollow part 212 can be set to 1.3 mm, and the inner diameter of the tip-end-side hollow part 213 can be set to 1.2 mm.

The base-end-side hollow part 211, the intermediate hollow part 212, and the tip-end-side hollow part 213 may have a tapered shape where the inner diameter gradually decreases toward the tip end.

While the locations in the axial direction of the first gradient change part 241 and the first stepped part 214 match, and the locations in the axial direction of the second gradient change part 242 and the second stepped part 215 match in the strain relief 202 described in the present example, the locations in the axial direction of each of the gradient change parts and the relevant stepped part may differ.

Also even when the inner shape of the hollow part is changed in a stepwise manner as in the present embodiment as described above, the same effect as that of the first embodiment can be achieved.

[Modified embodiment]

The strain reliefs described in the above embodiments have a configuration in which the outer diameter is changed so as to have a stepless (non-stepwise) outer shape. However, the strain relief may have a configuration in which the outer diameter is changed in such a manner that the outer shape becomes stepwise at least at one of the connection point between the outer diameter unchanged part 220 and the first outer-diameter gradually-decreasing part 231, and the connection point between the first outer-diameter gradually-decreasing part 231 and the second outer-diameter gradually-decreasing part 232.

In the above embodiments, the case in which the gradual decrease rate of the outer diameter in the first outer-diameter gradually-decreasing part 231 and the gradual decrease rate of the outer diameter in the second outer-diameter gradually-decreasing part 232 are uniform, and the gradual decrease rate of the outer diameter in the second outer-diameter gradually-decreasing part 232 is larger than the gradual decrease rate of the outer diameter in the first outer-diameter gradually-decreasing part 231 is described. That is, a shape in which the gradual decrease rate of the outer diameter changes so as to be increased in a stepwise manner toward the tip end is described in the above embodiments.

However, the outer-diameter gradually-decreasing part 230 may have a shape in which the gradual decrease rate of the outer diameter changes so as to be continuously increased toward the tip end. That is, the outer shape of the strain relief may be a shape in which the line of the outer circumferential surface illustrated in a vertical sectional view is curved.

While the inner diameter of the strain relief 201 is configured to be gradually decreased toward the tip end in the first embodiment, the inner diameter may be uniform in the axial direction.

The strain relief 202 described in the second embodiment has a configuration in which the inner diameter is changed in such a manner that the inner shape becomes stepped (stepwise). However, the strain relief may be configured to have a non-stepwise inner shape where the gradual decrease rate of the inner diameter is changed in a stepwise manner according to the locations in the axial direction. In the case in which the gradual decrease rate of the inner diameter is changed according to the locations in the axial direction, the gradual decrease rate of the inner diameter may be increased or decreased toward the tip end.

<Summary of action and effects of aspects of present invention>

<First aspect>

The present aspect is a catheter (an electrode catheter 100) comprising a hollow tube-shaped strain relief 200 including a hollow part 210 penetrating through in an axial direction, and a catheter body 10 having a base end part 10a inserted into the hollow part, wherein the strain relief includes an outer-diameter gradually-decreasing part 230 in which an outer diameter thereof gradually decreases toward a tip end, and a gradual decrease rate of the outer diameter in the outer-diameter gradually-decreasing part increases toward the tip end.
According to the present aspect, a catheter including a novel strain relief that prevents or suppresses kinks of the catheter body is provided.
Since the gradual decrease rate of the outer diameter in the outer-diameter gradually-decreasing part is increased toward the tip end in the strain relief used in the present aspect, the axial length of a region where the wall thickness is relatively small can be shortened. Therefore, damages such as break or split of the strain relief caused by the wall thickness reduction can be effectively prevented.

<Second aspect>

In the catheter (the electrode catheter 100) according to the present aspect, the outer-diameter gradually-decreasing part 230 includes a first outer-diameter gradually-decreasing part 231 on a base end side, and a second outer-diameter gradually-decreasing part 232 on a tip end side, the part 231 having an outer diameter that changes at a first gradual decrease rate and the part 232 having an outer diameter that changes at a second gradual decrease rate larger than the first gradual decrease rate.
Since the gradual decrease rate of the outer diameter in the outer-diameter gradually-decreasing part is increased toward the tip end in the strain relief used in the present aspect, the axial length of a region where the wall thickness is relatively small can be shortened. Therefore, damages such as break or split of the strain relief caused by the wall thickness reduction can be effectively prevented. Furthermore, since the gradual decrease rate of the outer diameter changes in a stepwise manner, a mold for strain relief molding can be easily formed.

<Third aspect>

The catheter (the electrode catheter 100) according to the present aspect is characterized in that an inner diameter of the hollow part 210 of the strain relief 200 gradually decreases from a base end to a tip end.
Due to the gradual decrease of the inner diameter of the hollow part 210 from the base end to the tip end, the decrease of the wall thickness in the outer-diameter gradually-decreasing part 230 is enabled to be gentle. Therefore, according to the present aspect, damages such as break or split of the strain relief caused by the wall thickness reduction are effectively prevented while the wall thickness of the outer-diameter gradually-decreasing part in the strain relief is ensured.

<Fourth aspect>

In the catheter (the electrode catheter 100) according to the present aspect, an outer diameter of the catheter body 10 is equal to or less than 1.0 mm.
The strain relief 200 according to the present aspect is particularly effective as means for preventing or suppressing kinks of an ultrafine catheter body of about equal to or less than 3 Fr (1.0 mm).

<Fifth aspect>

The catheter (the electrode catheter 100) according to the present aspect includes a handle 30 that supports a base end part 10a of the catheter body 10 inserted into the strain relief 200, wherein the strain relief has a housed part (at least a portion of an outer diameter unchanged part 220) on a base end side and an exposed part (at least the whole of the outer-diameter gradually-decreasing part 230) on a tip end side, the housed part being housed in the handle and the exposed part being exposed from the handle, and the gradual decrease rate of the outer diameter in the exposed part is larger than the gradual decrease rate of an inner diameter in the exposed part.

According to the present aspect, the gradual decrease rate of the outer diameter is set to be larger than that of the inner diameter in the exposed part. Therefore, while the wall thickness of the strain relief is reduced toward the tip end, the wall thickness changes gently. Therefore, the wall thickness required for preventing or suppressing kinks of the catheter body can be easily allocated in the exposed part. Furthermore, damages such as break or split of the strain relief caused by the wall thickness reduction can be effectively prevented.

Reference Signs List

100 electrode catheter (catheter), 10 catheter body, 10a base end part, 11 central lumen, 20 ring-shaped electrode, 30 handle, 31 first through hole, 31a support part, 31b guide wire path, 31c intermediate portion, 32 second through hole, 32a tip end, 32b the other end, 35, 35A divided piece, 36 coupling protrusion, 37 first recess, 38 second recess, 40 guide wire, 50 electrode connector, 200, 201, 202 strain relief, 210 hollow part, 211 base-end-side hollow part, 212 intermediate hollow part, 213 tip-end-side hollow part, 214 first stepped part, 215 second stepped part, 220 outer diameter unchanged part, 230 outer-diameter gradually-decreasing part, 231 first outer-diameter gradually-decreasing part, 232 second outer-diameter gradually-decreasing part, 241 first gradient change part, 242 second gradient change part

**Claims**

1. A catheter comprising a hollow tube-shaped strain relief including a hollow part penetrating through in an axial direction, and a catheter body having a base end part inserted into the hollow part, wherein
   the strain relief includes an outer-diameter gradually-decreasing part in which an outer diameter thereof gradually decreases toward a tip end, and a gradual decrease rate of the outer diameter in the outer-diameter gradually-decreasing part increases toward the tip end.

2. The catheter according to claim 1, wherein the outer-diameter gradually-decreasing part includes a first outer-diameter gradually-decreasing part on a base end side, and a second outer-diameter gradually-decreasing part on a tip end side, the first outer-diameter gradually-decreasing part having an outer diameter that changes at a first gradual decrease rate and the second outer-diameter gradually-decreasing part having an outer diameter that changes at a second gradual decrease rate larger than the first gradual decrease rate.

3. The catheter according to claim 1 or 2, wherein an inner diameter of the hollow part gradually decreases from a base end to a tip end.

4. The catheter according to any one of claims 1 to 3, wherein an outer diameter of the catheter body is equal to or less than 1.0 millimeter.

5. The catheter according to any one of claims 1 to 4, including a handle that supports the base end part of the catheter body inserted into the strain relief, wherein
   the strain relief has a housed part on a base end side and an exposed part on a tip end side, the housed part being housed in the handle and the exposed part being exposed from the handle, and a gradual decrease rate of the outer diameter in the exposed part is larger than a gradual decrease rate of an inner diameter in the exposed part.

# FIG.1(a)

# FIG.1(b)

# FIG.2

35(35A)

37

38

36

36

36

36

36

36

37

BASE END PART

TIP END PART

# FIG.3

# FIG.4

## INTERNATIONAL SEARCH REPORT

| International application No. |
| --- |
| PCT/JP2019/038703 |

A. CLASSIFICATION OF SUBJECT MATTER
Int.Cl. A61M25/01(2006.01)i

According to International Patent Classification (IPC) or to both national classification and IPC

B. FIELDS SEARCHED

Minimum documentation searched (classification system followed by classification symbols)
Int.Cl. A61M25/01

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y | US 5507732 A (MEDTRONIC, INC.) 16 April 1996, column 3, line 55 to column 4, line 14, fig. 1-2 (Family: none) | 1-3<br>4-5 |
| X<br>Y | US 5941849 A (SCIMED LIFE SYSTEMS, INC.) 24 August 1999, column 4, lines 5-55, fig. 1-2 & WO 1999/010039 A1 | 1-3<br>4-5 |
| Y | US 2007/0005001 A1 (ABBOTT LABORATORIES) 04 January 2007, paragraph [0065], fig. 1-2 & WO 2007/005584 A1 | 1-3<br>4-5 |

☒ Further documents are listed in the continuation of Box C.  ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 22 November 2019 (22.11.2019) | 03 December 2019 (03.12.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

International application No.

PCT/JP2019/038703

**INTERNATIONAL SEARCH REPORT**

C (Continuation).    DOCUMENTS CONSIDERED TO BE RELEVANT

| Category* | Citation of document, with indication, where appropriate, of the relevant passages | Relevant to claim No. |
|---|---|---|
| Y | GB 2538124 A (DIASOLVE LTD.) 09 November 2016, page 8, line 11 to page 12, line 4, fig. 1-2, 5 & WO 2017/098198 A1 | 4-5 |
| Y | US 5666970 A (HEART RHYTHM TECHNOLOGIES, INC.) 16 September 1997, column 7, line 10 column 8, line 41, fig. 4-5 & WO 1996/034650 A1 | 5 |

Form PCT/ISA/210 (continuation of second sheet) (January 2015)

**EP 4 039 314 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018143603 A